# EUROPEAN PATENT APPLICATION

(11) **EP 4 748 411 A2**
(43) Date of publication of application: **27.05.2026**
(21) Application number: 26163260.8
(22) Date of filing: 19.06.2018
(51) Int. Cl.: A61M 5/162

(54) **NEEDLELESS IV INJECTION PORT**

(30) Priority: 21.06.2017 US 201715629000; 21.06.2017 US 201715628990; 21.02.2018 US 201815901741
(62) Divisional of application: 18820792.2
(71) Applicant: Fresenius Kabi Deutschland GmbH, 61352 Bad Homburg (DE)
(72) Inventor: RYAN, Dana Wm., Mt. Juliet, 37122 (US); RYHERD, Anthony E., Austin, 78737 (US); KAISER, James M., Austin, 78739 (US)
(74) Representative: Fresenius Kabi Deutschland GmbH

(57) **Abstract**

An injection port assembly comprises a body having a first mating structure and a second mating structure coupled together. A resilient barrier is received within the body and compressible between a less compressed first position in which fluid flow through the injection port assembly is blocked, to a more compressed second position in which fluid flow through the injection port assembly is permitted. A hollow cannula is coupled to the first mating structure and configured to be received within the resilient barrier. The hollow cannula has a distal end portion configured to extend through the resilient barrier when the resilient barrier is in the more compressed second position.

## Description

### CROSS-REFERENCE TO RELATED APPLICATIONS

This application claims priority to U.S. Patent Application Serial No. 15/628,990 filed June 21, 2017, U.S. Patent Application Serial No. 15/629,000 filed June 21, 2017, and U.S. Patent Application Serial No. 15/901,741, filed February 21, 2018, the entire contents of which are incorporated herein by reference.

### BACKGROUND OF THE INVENTION

### 1. Field of the Invention

The present disclosure relates generally to medical intravenous (IV) administration line connectors. More particularly, this disclosure pertains to a needleless, intermittent, injection port for safe infusion of IV fluids, antibiotics, lipids, blood, blood components or drug products and/or blood aspiration in intravenous and blood administration therapy.

### 2. Description of the Prior Art

One important aspect of needleless injection ports is the ability of the medical worker using the injection port to visualize the fluids flowing through the injection port. This is important for several reasons. First, during the primary usage of the injection port it may be important to visualize the proper flow of fluids through the injection port. After the primary usage, it may be important to visualize the passages through the injection port during the flushing of the injection port. Finally, during all usages of the injection port it is important to be able to detect any malfunction of the injection port, such as leakage between its various components.

These issues have led to the development of so-called "clear" injection ports, wherein all of the components of the injection port are transparent, thus allowing visualization of the fluids in the injection port.

There is a continuing need for improvements in such injection ports to make the same more reliable in use, and to make malfunctions more readily detectable.

### SUMMARY OF THE INVENTION

In one aspect, an injection port assembly may include a body having first and second mating structures configured to be coupled together. A resilient barrier is configured to be received within the body and is compressible from a less compressed first position in which fluid flow through the injection port assembly is blocked, to a more compressed second position in which fluid flow through the injection port assembly is permitted. The resilient barrier includes an internal cavity. A hollow cannula is coupled to the first mating structure and is configured to be received within the resilient barrier. The hollow cannula has a cannula distal end portion configured to extend through the resilient barrier when the resilient barrier is in the more compressed second position. The cannula distal end portion may have at least one outlet window.

At least a portion of the hollow cannula may be opaque, and at least a portion of both the second mating structure and the resilient barrier may be transparent, so that any fluid leakage into the internal cavity of the resilient barrier is visible through the resilient barrier and the second mating structure against an opaque background provided by the opaque cannula.

The entire cannula may be opaque, and the entire resilient barrier and second mating structure may be transparent.

In one embodiment, the injection port assembly may be particularly adapted for visualization of blood leakage, and the opaque cannula may have a color that is lighter than blood so as to provide a contrasting background to any blood leakage outside of the cannula.

The opaque cannula may have a color other than red, so as to contrast to any blood leakage outside of the cannula.

The opaque cannula may have a pastel color so as to contrast with blood in the internal cavity of the resilient barrier.

In one embodiment, the opaque cannula may be colored a light pastel blue.

The opaque cannula may be integrally formed with the first mating structure and the entire first mating structure may be opaque.

The hollow cannula has an internal fluid passageway, and the cannula is sufficiently opaque that blood inside the internal fluid passageway is not visible through the cannula.

In another aspect, an injection port assembly may include a body having a first mating structure and a second mating structure configured to be coupled to the first mating structure. A resilient barrier may be configured to be received within the body and compressible from a less compressed first position in which fluid flow through the injection port assembly is blocked, to the more compressed second position in which fluid flow through the injection port assembly is permitted. The resilient barrier may include an internal cavity. A hollow cannula may be coupled to the first mating structure and configured to be received within the internal cavity of the resilient barrier. The hollow cannula may have a cannula end portion configured to extend through the resilient barrier when the resilient barrier is in the more compressed second position. The cannula may have an internal fluid passageway. At least a portion of the hollow cannula is opaque so that the internal fluid passageway of the cannula is not visible through the cannula. At least a portion of both the second mating structure and the resilient barrier may be transparent so that the internal cavity of the resilient barrier is visible through the resilient barrier and the second mating structure.

In certain embodiments of any aspect described herein, the internal cavity, when the resilient barrier is in a relaxed state, includes a cavity nose portion, a cavity sealing portion, and a cavity guide portion. The cavity nose portion has a cavity nose portion maximum inside diameter. The cavity sealing portion has a cavity sealing portion length, the cavity sealing portion having a cavity sealing portion inside diameter smaller than the cavity nose portion inside diameter along at least a majority of the cavity sealing portion length. The cavity guide portion is located on an opposite side of the cavity sealing portion from the cavity nose portion. The cavity guide portion has a cavity guide portion inside diameter greater than the cavity sealing portion inside diameter.

In some such embodiments, the cannula distal end portion has at least one lateral outlet window having a window length less than the cavity sealing portion length.

In some such embodiments, the cannula distal end portion includes a cannula nose located distally of the lateral outlet window and configured to be closely received in the cavity nose portion of the resilient barrier when the resilient barrier is in the less compressed first position. The cannula distal end portion both distally and proximally of the lateral outlet window has a cannula distal end portion outside diameter sufficiently greater than the cavity sealing portion inside diameter such that when the cannula is received in the resilient barrier with the cannula nose received in the cavity nose portion there is an interference fit between the cannula and the resilient barrier extending along the lateral outlet window and both proximally and distally of the lateral outlet window so that the cavity sealing portion of the resilient barrier seals across the lateral outlet window.

The cavity nose portion may be bulbous in shape and may have a semi-spherical distal end. The cavity nose portion may include a frusto-conical portion of increasing diameter in a proximal direction from the semi-spherical distal end to the cavity nose portion maximum inside diameter.

The cavity sealing portion may include a frusto-conical portion of increasing diameter in a proximal direction from a cavity sealing portion minimum inside diameter to a cavity sealing portion maximum inside diameter.

The cavity guide portion may include a frusto-conical portion of increasing diameter in a proximal direction from the cavity sealing portion.

The interference fit between the cannula and the resilient barrier may extend into the frusto-conical portion of the cavity guide portion.

The cavity guide portion may include a first frusto-conical portion of increasing diameter in a proximal direction adjacent the cavity sealing portion and a second frusto-conical portion adjacent the first frusto-conical portion, the second frusto-conical portion having a smaller included angle than the first frusto-conical portion.

The interference fit between the cannula and the resilient barrier may extend at least about 0.010 inch proximally and distally of the outlet window.

The cannula and the resilient barrier may be configured such that the interference fit provides at least about 0.001 inch radial interference between the cannula and the resilient barrier. Optionally the interference fit may provide at least about 0.002 inch radial interference. Optionally the interference fit may provide at least about 0.004 inch radial interference. Optionally the interference fit may provide at least about 0.006 inch radial interference.

The cannula nose may substantially fill the cavity nose portion when the resilient barrier is in the less compressed first position with the cannula nose closely received in the cavity nose portion.

When the resilient barrier is in a relaxed state the cavity sealing portion inside diameter may be smaller than the cavity nose portion maximum inside diameter along the entire cavity sealing portion length.

The at least one lateral outlet window in one embodiment includes two diametrically opposed outlet windows, and in another embodiment includes three circumferentially equally spaced outlet windows.

In certain embodiments of any aspect described herein, the hollow cannula may have a longitudinal central axis. In some such embodiments, the cannula further includes at least one lateral outlet window formed in the cannula distal end portion, the at least one lateral outlet window having a window width perpendicular to the longitudinal central axis.

In certain embodiments of any aspect described herein, an internal fluid passageway is defined in the hollow cannula and configured to communicate the at least one lateral outlet window with a fluid conduit connected to the first mating structure. The internal fluid passageway may have a non-circular cross section axially proximal from the window. The non-circular cross section may have a cross section area greater than a cross section area of a circle of diameter equal to the window width.

The at least one lateral outlet window may comprise two diametrically opposed outlet windows.

The two diametrically opposed outlet windows may be diametrically spaced apart by a window spacing.

The internal fluid passageway may extend laterally to each of the two diametrically opposed outlet windows and the non-circular cross section may have a first lateral cross section dimension at least equal to the window spacing immediately adjacent a proximal end of the windows.

The non-circular cross section immediately adjacent the proximal ends of the windows may have a second lateral cross section dimension perpendicular to the first lateral cross section dimension, which second lateral cross section dimension is at least equal to the window width.

The non-circular cross section of the internal fluid passageway may be at least partially defined between first and second generally parallel opposed interior walls of the hollow cannula.

The first and second interior walls may extend along a length of the windows.

The hollow cannula may further include first and second diametrically opposed reinforcing ribs extending radially inward from the first and second opposed interior walls, respectively, along at least the length of the windows.

The first and second reinforcing ribs may continue proximally beyond the length of the windows further into the internal fluid passageway.

The at least one lateral outlet window may comprise three circumferentially equally spaced outlet windows.

The non-circular cross section of the internal fluid passageway may be a three lobed cross section.

The three lobed cross section may taper radially outward and extend proximally from the windows for a distance at least as long as the window length.

The at least one window may have a window length, and the non-circular cross section of the internal fluid passageway may extend proximally beyond the length of the window further into the internal fluid passageway by a further distance at least as long as the window length.

In certain embodiments of any aspect described herein, the injection port assembly may include a snap lock feature for locking the first and second mating structures together. The snap lock feature may include a first locking portion and a second locking portion. One of the first and second locking portions may include a locking edge and the other of the first and second locking portions may include a tapered locking surface. The locking edge is configured to engage the tapered locking surface to resist disengagement of the first and second mating structures.

The locking edge may be defined by a substantially 90 degree corner.

The tapered locking surface may be a curved tapered locking surface.

The tapered locking surface may be defined on the second locking portion of the second mating structure, and the tapered locking surface may be a segmented surface defined on a plurality of stabilizing ring securement segments of the second mating structure.

The first and second locking portions may be configured such that a force of at least 30 pounds, and more preferably at least 40 pounds, is required to pull apart the first and second mating structures.

Numerous objects, features and advantages of the present invention will really be readily apparent to those skilled in the art upon reading of the following disclosure when taken in conjunction with the accompanying drawings.

### BRIEF DESCRIPTION OF THE DRAWINGS

Figure 1 is an exploded view of the components of the injection port assembly.
Figure 2 is an assembled elevation view of the injection port assembly of Figure 1.
Figure 3 is an elevation cross-section view of the injection port assembly of Figures 1 and 2 taken along line 3-3 of Figure 2.
Figure 4 is an elevation cross-section view of the injection port assembly of Figure 1 in the less compressed position of the resilient barrier. A syringe is shown in position about to be engaged with the injection port assembly.
Figure 5 is a view similar to Figure 4, the syringe having been engaged with the injection port assembly to move the resilient barrier to its more compressed second position in which fluid flow through the injection port assembly is permitted.
Figure 6 is an elevation view of a second embodiment of the lower body portion having three lateral outlet windows.
Figures 6A-6D are cross section views taken along lines 6A-6A, 6B-6B, 6C-6C and 6D-6D of Figure 6.
Figure 7 is an enlarged view of the distal end portion of the cannula of the lower body portion of Figure 6.
Figure 8 is an elevation view of a second embodiment of the resilient barrier, for use with the lower body portion of Figure 6.
Figure 9 is an enlarged cross section view of the distal end portion of the resilient barrier of Figure 8.
Figure 10 is an elevation cross section view of an injection port assembly using the lower body portion and resilient member of Figures 6 and 8 in the less compressed position of the resilient barrier. A syringe is shown in position about to be engaged with the injection port assembly.
Figure 11 is a view similar to Figure 10, the syringe having been engaged with the injection port assembly to move the resilient barrier to its more compressed second position in which fluid flow through the injection port assembly is permitted.
Figure 12 is an elevation view of a Y-site injection port assembly including the resilient member and cannula construction of Figures 6-11.
Figure 13 is an elevation cross section view of the Y-site injection port assembly of Figure 12.
Figure 14 is an elevation cross section view of an embodiment of the injection port assembly.
Figure 15 is an elevation view of the first mating structure of the body, including the hollow cannula.
Figure 16 is an elevation cross section view of the first mating structure and cannula taken along line 16-16 of Figure 15.
Figure 17 is a top plan view of the first mating structure and cannula of Figure 15.
Figure 18 is an elevation view of the first mating structure and cannula of Figure 15, rotated 90° about the longitudinal axis as compared to Figure 15.
Figure 19 is an enlarged view of the distal end portion of the cannula circled in Figure 18.
Figure 20 is a cross section view taken along line 20-20 of Figure 18 showing the non-circular cross section of the internal fluid passageway of the cannula.
Figure 21 is an elevation view of the resilient barrier of the injection port assembly of Figure 14, with an internal cavity of the resilient barrier indicated in dashed lines.
Figure 22 is an enlarged elevation cross section view of the upper portion of the resilient barrier of Figure 21.
Figure 23 is an elevation view of the second mating structure or upper body portion of the injection port assembly of Figure 14.
Figure 24 is an elevation cross section view of the upper body portion of Figure 23.
Figure 25 is an exploded view of the components of an alternative embodiment of the injection port assembly.
Figure 26 is an assembled elevation cross-section view of the injection port assembly of Figure 25.

### DETAILED DESCRIPTION

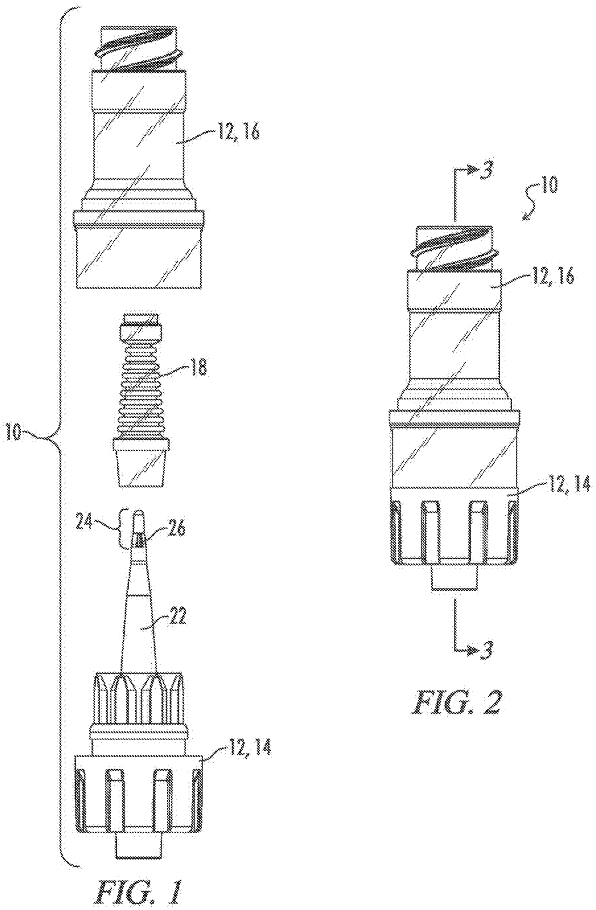

Referring now to the drawings, and particularly to Figures 1 and 2, an injection port assembly is shown and generally designated by the number 10. The injection port assembly 10 includes a body 12 having a first mating structure 14 and a second mating structure 16 configured to be coupled to the first mating structure 14.

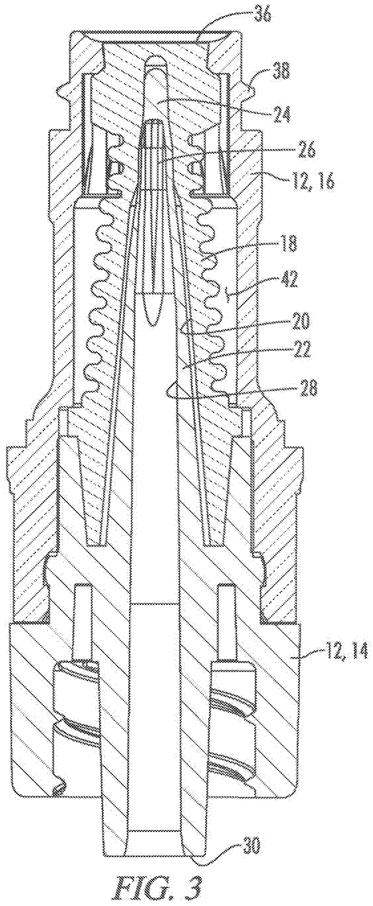

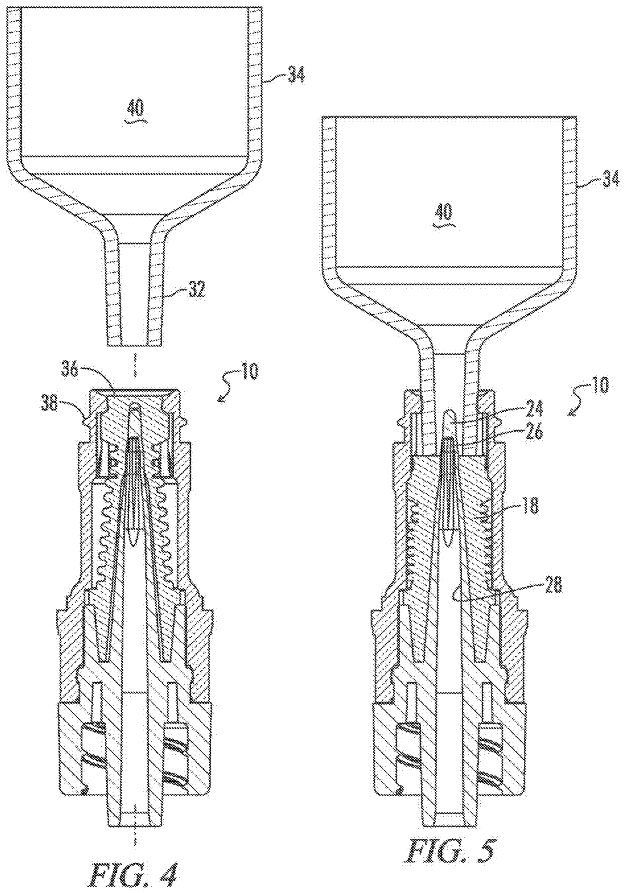

A resilient barrier 18 is configured to be received within the body 12 and is compressible from a less compressed first position as seen in Figure 4, in which fluid flow through the injection port assembly 10 is blocked, to a more compressed second position as seen in Figure 5, in which fluid flow through the injection port assembly 10 is permitted. The resilient barrier 18 has an internal cavity 20, as seen in Figure 3.

A hollow cannula 22 is coupled to the first mating structure 14 and configured to be received within the resilient barrier 18. The hollow cannula 22 has a cannula distal end portion 24 configured to extend through the resilient barrier 18 when the resilient barrier 18 is in the more compressed second position as seen in Figure 5. The cannula distal end portion 24 has at least one outlet window 26 defined therein.

The hollow cannula 22 is preferably integrally formed with the first mating structure 14. Hollow cannula 22 has an internal fluid passageway 28 defined therein, communicating a proximal end 30 of the first mating structure 14 with the outlet windows 26.

As illustrated in Figures 4 and 5, the connector 10 is constructed to be engaged by a male Luer tip 32 of a syringe 34 or other medical component. The male Luer tip 32 engages the distal end 36 of resilient barrier 18 and forces the same downward over the hollow cannula 22 so that the distal portion 24 thereof is exposed, allowing fluid communication between the syringe 34 and the windows 26 of the internal fluid passageway 28 of cannula 22.

The syringe 34 may also include a Luer lock connection (not shown) having female threads which engage the male threads 38 of the second mating structure 16.

As will be appreciated in viewing Figure 5, when the syringe 34 is engaged with the injection port assembly 10 and moves the resilient barrier 18 to its more compressed second position, fluid communication is provided between the interior 40 of the syringe 34 and the internal fluid passageway 28 of the hollow cannula 22. This allows fluid to either flow out of the syringe 34 and through the internal fluid passageway 28 to other components of the intravenous assembly to move fluids into a patient 's body, or fluids such as blood may be drawn from the patient 's body upward through the internal fluid passageway 28 into the interior 40 of the syringe 34. When fluid flows in either direction a properly functioning injection port assembly 10 will not have any fluid leakage outside of the fluid pathway just described.

In the event of undesired leakage, it is possible that fluids may find their way into the internal cavity 20 of the resilient barrier 18, or into an annulus 42 between the resilient barrier 18 and the second mating structure for 16. The present invention is particularly adapted for the ease of visualization and detection of such leakage into either the internal cavity 20 of resilient barrier 18 or into the annulus 42 between the resilient barrier 18 and the second mating structure 16. This is accomplished by making at least a portion of the hollow cannula 22, and preferably the entire cannula 22 and the entire first mating structure 14, from an opaque material so that the internal fluid passageway 28 is not visible through the opaque cannula 22. Particularly fluids such as blood contained in the internal fluid passageway 28, should not be visible through the opaque cannula 22. Furthermore, at least a portion of the resilient barrier 18 and at least a portion of the second mating structure 16 of body 12 are made from transparent materials so that the internal cavity 20 of the resilient barrier 18 and the exterior surface of cannula 22 are readily visible through the second mating structure 16 and the resilient barrier 18. Preferably the entire resilient barrier 18 and the entire second mating structure 16 are made from transparent materials.

Thus any fluid leakage into the internal cavity 20 of resilient barrier 18 is readily visible through the resilient barrier 18 and the second mating structure 16 against the opaque background provided by the exterior surface of the opaque cannula 22.

This is contrasted to the situation in the so called "clear" injection ports of the prior art in which all components including the cannula are clear. If the cannula is also clear, then for example when withdrawing blood from a patient there is always blood visible in the internal fluid passageway 28 of the cannula 22 and thus it can be difficult to visualize blood leakage into the internal cavity 20 of the resilient barrier 18.

Depending upon the fluids which are expected to be transmitted through the injection port assembly 10, the color of the opaque cannula 22 may be selected so as to provide the greatest contrast for ease of visualization of any fluids which might leak into the internal cavity 20 of resilient barrier 18 or into the annulus 42.

For example, when the injection port assembly 10 is anticipated to be used for drawing blood samples from the patient, and when it is desired to be able to visualize any blood leakage, the color of the opaque cannula 22 may be selected so as to provide a distinct contrast with any blood which leaks into the internal cavity 20. For example, the opaque cannula 22 may have a color which is lighter than blood. Preferably in this situation the opaque cannula 22 will have a color other than red. The opaque cannula 22 may have a pastel color such as to contrast with blood in the internal cavity 20. The cannula 22 in a preferred embodiment may be colored a light pastel blue, which provides a distinct contrast with any blood leakage into the internal cavity 20. A white colored opaque cannula 22 also provides a good contrasting background to blood.

One particularly suitable light pastel blue color is Pantone PMS-544. Another suitable light pastel blue color is Pantone PQ - 7541C. Other suitable opaque pastel background colors, depending upon the fluid desired to be visualized, may include:
Pantone 11-0603 Pastel Parchment;
Pantone 11-0616 Pastel Yellow
Pantone 12-1007 Pastel Rose Tan
Pantone 12-4607 Pastel Blue
Pantone 13-0116 Pastel Green
Pantone 13-6309 Pastel Turquoise.

If the injection port assembly is to be optimized for visualization of specific fluids to be injected into the patient's body, then a suitable contrasting opaque color for the cannula 22 may be selected.

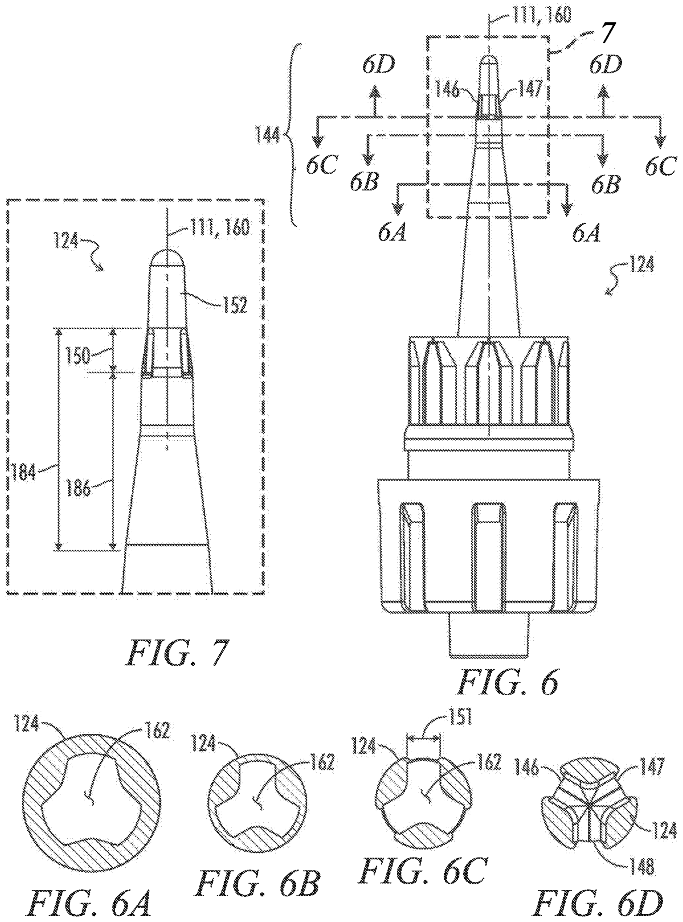

### The Embodiments of Figures 6-11:

An embodiment of an injection port assembly having three lateral outlet windows is shown in FIGS. 6-11 and is generally designated by the number 110.

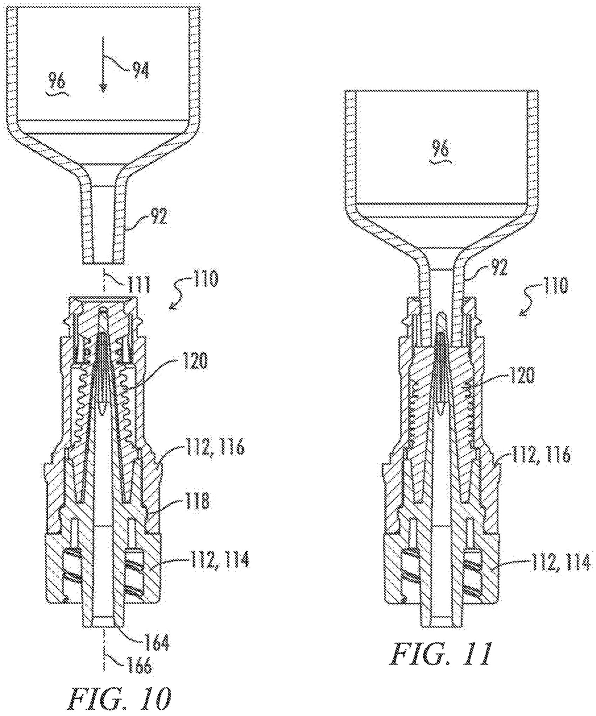

The injection port assembly 110 has a longitudinal axis 111. The injection port assembly 110 is shown in assembled cross section in FIG. 10 and includes a body 112 made up of a first mating structure 114 and a second mating structure 116. The first mating structure 114 may also be referred to as a lower body part 114, and the second mating structure 116 may also be referred to as an upper body part 116. The first and second mating structures 114 and 116 are coupled together by a snap lock feature 118.

The injection port assembly 110 further includes a resilient barrier 120 which is configured to be received within the body 112 and which is compressible from a less compressed first position as seen for example in FIG. 10, in which fluid flow through the injection port assembly 110 is blocked, to a more compressed second position as seen for example in FIG. 11, in which fluid flow through the injection port assembly 110 is permitted.

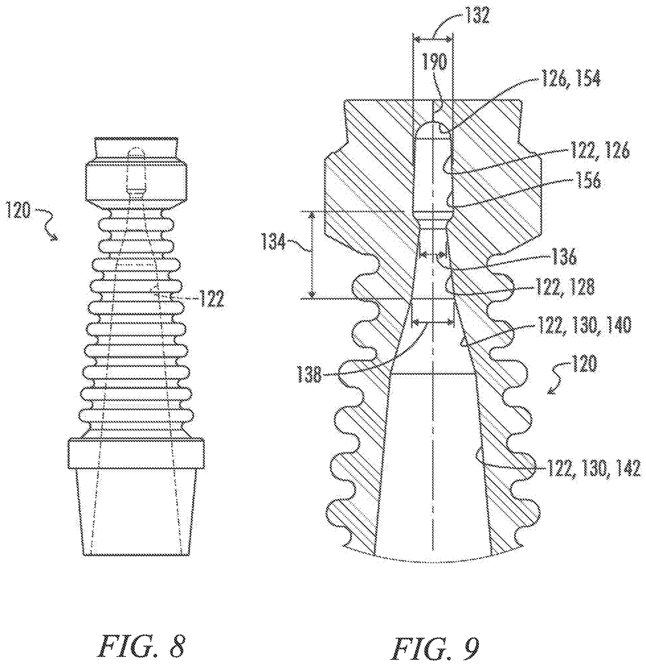

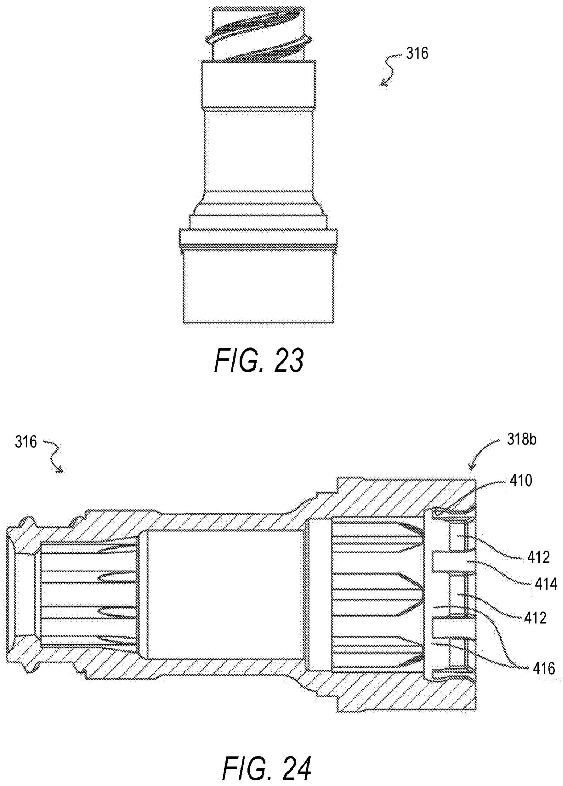

The details of construction of the first mating structure 114 are best shown in FIGS. 6-7. The details of construction of the second mating structure 116 are substantially the same as shown for the second mating structure 316 in FIGS. 23 and 24. The details of construction of the resilient barrier 120 are best shown in FIGS. 8 and 9.

FIG. 10 shows the injection port assembly 110 in an assembled cross section view with the first and second mating structures 114 and 116 coupled together and with the resilient barrier 120 received within the body 112 between the first and second mating structures 114 and 116.

As best seen in FIGS. 8 and 9, the resilient barrier 120 includes an internal cavity 122. It will be appreciated that the resilient barrier 120 is formed from an elastomeric material, and is shown in FIGS. 8 and 9 in its relaxed state in which the elastomeric material is relatively undeformed. It will also be appreciated that in FIG. 10 a hollow cannula 124 of the first mating structure 114 has been received in the internal cavity 122, thus deforming portions of the resilient barrier 120 radially outward so that the shape of the resilient barrier 120 as seen in FIG. 10, and particularly of its internal cavity 122, are different due to the resilient deformation thereof.

Referring now to FIGS. 8 and 9 which show the resilient barrier 120 and particularly its internal cavity 122 in their relaxed state, the internal cavity 122 in this relaxed state may be described as including a cavity nose portion 126, a cavity sealing portion 128, and a cavity guide portion 130.

As shown in FIG. 9, the cavity nose portion 126 has a cavity nose portion maximum inside diameter 132.

The cavity sealing portion 128 may be described as having a cavity sealing portion length 134. The cavity sealing portion 128 has a minimum cavity sealing portion inside diameter 136 at its upper end and is slightly tapered to a maximum cavity sealing portion inside diameter 138 at its lower end. It is noted that the cavity sealing portion 128 overall can be described as having a cavity sealing portion inside diameter smaller than the cavity nose portion maximum inside diameter 132 along at least a majority of the cavity sealing portion length 134. The cavity sealing portion inside diameter may be smaller than the cavity nose portion maximum inside diameter 132 along substantially the entire cavity sealing portion length 134.

The cavity sealing portion 128 of internal cavity 122 may be described as including a frusto-conical portion of increasing diameter in a proximal direction which increases from cavity sealing portion minimum inside diameter 136 to cavity sealing portion maximum inside diameter 138.

The cavity nose portion 126 may be described as being bulbous in shape as seen best in FIG. 9, and having a semi-spherical distal end 154. The cavity nose portion 126 may be further described as including a frusto-conical portion 156 of increasing diameter in a proximal direction from the semi-spherical distal end 154 to the cavity nose portion maximum inside diameter 132.

The cavity guide portion 130 is located on an opposite side of the cavity sealing portion 128 from the cavity nose portion 126. The cavity guide portion 130 tapers radially outward from the cavity sealing portion 128 and thus may be described as having a cavity guide portion inside diameter greater than the cavity sealing portion inside diameter 138. The cavity guide portion 130 may be further described as including a first frusto-conical portion 140 of increasing diameter in a proximal direction from the cavity sealing portion 138, and a second frusto-conical portion 142 adjacent the first frusto-conical portion 140, the second frusto-conical portion 142 having a smaller included angle than the first frusto-conical portion 140.

As previously noted, a hollow cannula 124 is coupled to the first mating structure 114, and in the example illustrated, the hollow cannula 124 is integrally formed with the first mating structure 114. The hollow cannula 124 is configured to be received within the resilient barrier 120 as shown for example in FIG. 10.

The hollow cannula 124 includes a distal end portion 144 shown in enlarged view in FIG. 7. The distal end portion 144 is configured to extend through the resilient barrier 120 when the resilient barrier 120 is in the more compressed second position of FIG. 11. The cannula distal end portion 144 has at least one lateral outlet window 146 and in the example shown has three lateral outlet windows 146, 147 and 148.

As seen in FIGS. 6 and 7, each of the lateral outlet windows 146, 147 and 148 has a window length 150 which is less than the cavity sealing portion length 134. As best seen in FIG. 6C, the windows 146, 147 and 148 also have a width 151 perpendicular to the longitudinal central axis 111, 160 of the injection port assembly 110 and the cannula 124.

The cannula distal end portion 144 includes a cannula nose 152 located distally of the lateral outlet windows 146, 147 and 148, and configured to be closely received in the cavity nose portion 126 of the resilient barrier 120 when the resilient barrier 120 is in the less compressed first position as shown in FIG. 10.

The cannula distal end portion 144 has a cannula distal end portion outside diameter both distally and proximally of the lateral outlet windows 146, 147 and 148, which cannula distal end portion outside diameter is sufficiently greater than the respective inside diameters of the cavity sealing portion 128 of internal cavity 122 of resilient barrier 120 when the cannula nose 152 is received in the cannula nose portion 126 such that there is an interference fit between the cannula 124 and the resilient barrier 120. The interference fit extends along the lateral outlet windows 146, 147 and 148 and both proximally and distally of the lateral outlet windows 146, 147 and 148 so that the cavity sealing portion 128 of the resilient barrier 120 seals across the lateral outlet windows 146, 147 and 148.

Preferably the radial interference along the interference fit is at least about 0.001 inch, optionally at least about 0.002 inch, optionally at least about 0.004 inch and optionally at least about 0.006 inch. Preferably the interference fit between the cannula 124 and the resilient barrier 120 extends at least about 0.010 inch both proximally and distally from the lateral outlet windows 146, 147 and 148.

The hollow cannula 124 has a longitudinal central axis 160 which is coincident with the central axis 111 of the injection port assembly 110.

The cannula 124 has an internal fluid passageway 162 defined therein as best seen in FIGS. 6A-6C. The internal fluid passageway 162 communicates the lateral outlet windows 146, 147 and 148 with a proximal end of the first mating structure 114 which is configured to communicate with a fluid conduit 166 schematically illustrated in FIG. 10. The fluid conduit 166 may be representative of any structure to which the injection port assembly 110 is to be connected for fluid flow therewith.

FIG. 6D shows an upward facing cross section of the cannula 124 taken along line D-D of FIG. 6. FIGS. 6A, 6B and 6C show downward facing cross sections of the cannula 124 taken along lines A-A, B-B and C-C, respectively. FIGS. 6A-6C show that the internal fluid passageway 162 has a non-circular cross section axially proximal from the windows 146, 147 and 148. This non-circular cross section may be described as having a cross sectional area greater than a cross section area of a circle of diameter equal to the window width 151.

The three outlet windows 146, 147 and 148 may be described as being equally circumferentially spaced about the axis 160. The non-circular cross section internal fluid passageway 162 as seen in FIGS. 6A-6D may be described as extending laterally to each of the outlet windows 146, 147 and 148.

The non-circular cross section of internal fluid passageway 162 may be described as a three lobed cross section. As can be seen in comparing FIGS. 6A, 6B and 6C, the three lobed cross section tapers radially outward. This cross sectional shape of the internal fluid passageway 62 as visually depicted in FIGS. 6A-6C preferably extends along a non-circular cross section length 184 shown in FIG. 7. As is apparent in FIG. 7, the non-circular cross section length 184 extends along the window length 150 and continues proximally beyond the window length 150 into the internal passageway 162 of cannula 124 by a further distance 186 at least as long as the window length 150 and preferably longer than the window length 150. Proximally of the non-circular cross sectional length 184, the internal passageway 162 may transition into a circular cross section extending to the proximal end 164 of the first mating structure 114.

At least a portion of the hollow cannula 124, and preferably the entire hollow cannula 124 and the entire first mating structure 114, are constructed from an opaque material so that the internal fluid passageway 162 is not visible through the opaque cannula 124. For example, fluids such as blood contained in the internal fluid passageway 162, should not be visible through the opaque cannula 124. Furthermore, at least a portion of the resilient barrier 120 and at least a portion of the second mating structure 116 of body 112 are made from transparent materials so that the internal cavity 122 of the resilient barrier 120 and the exterior surface of cannula 124 are readily visible through the second mating structure 116 and the resilient barrier 120. Preferably the entire second mating structure 116 and the entire resilient barrier 120 are made from transparent material.

In a particular embodiment, at least a portion of the hollow cannula 124 is opaque, at least a portion of the second mating structure 116 is transparent, and at least a portion of the resilient barrier 120 is semi-transparent (e.g., translucent). Preferably, the entire hollow cannula 124 is a pastel opaque color, the entire second mating structure 116 is transparent, and the entire resilient barrier 120 is semi-transparent (e.g., translucent).

Thus any fluid leakage into the internal cavity 122 of resilient barrier 120 is readily visible through the resilient barrier 120 and the second mating structure 116 against the opaque background provided by the exterior surface of the opaque cannula 124.

Depending upon the fluids which are expected to be transmitted through the injection port assembly 110, the color of the opaque cannula 124 may be selected so as to provide the greatest contrast for ease of visualization of any fluids which might leak into the internal cavity 122 of resilient barrier 120. For example, in certain embodiments, the pastel color is selected from the group consisting of light blue, blue, green, and turquoise. In one particular embodiment, the cannula 124 may be colored a light pastel blue, which provides a distinct contrast with any blood leakage into the internal cavity 122.

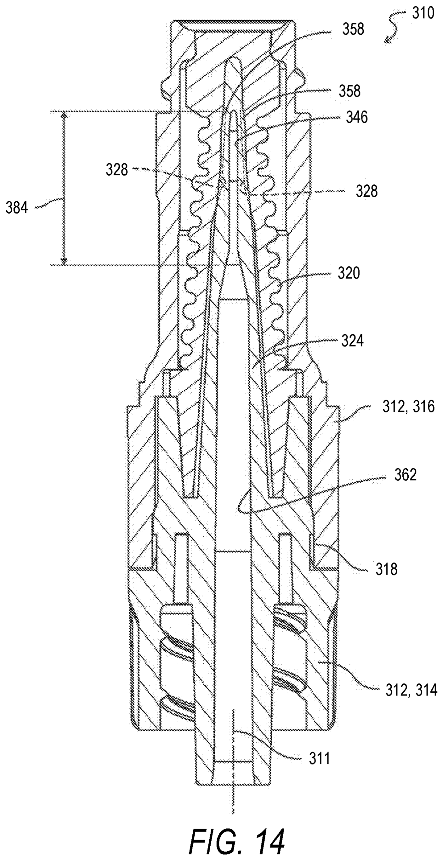

### The Embodiment of FIGS. 14-24:

Referring now to the drawings, and particularly to FIG. 14, an embodiment of an injection port assembly is shown and generally designated by the numeral 310. The injection port assembly 310 has a longitudinal axis 311. The injection port assembly 310 includes a body 312 made up of a first mating structure 314 and a second mating structure 316. The first mating structure 314 may also be referred to as a lower body part 314, and the second mating structure 316 may also be referred to as an upper body part 316. The first and second mating structures 314 and 316 are coupled together by a snap lock feature 318.

The injection port assembly 310 further includes a resilient barrier 320 which is configured to be received within the body 312 and which is compressible from a less compressed first position as seen for example in FIG. 14, in which fluid flow through the injection port assembly 310 is blocked, to a more compressed second position in which fluid flow through the injection port assembly 310 is permitted. It is noted that FIGS. 10 and 11 illustrate a similar less compressed first position and more compressed second position for the alternative embodiment of FIGS. 6-11, and FIGS. 10 and 11 are also representative of the change in shape of the resilient barrier 320 for the injection port assembly 310.

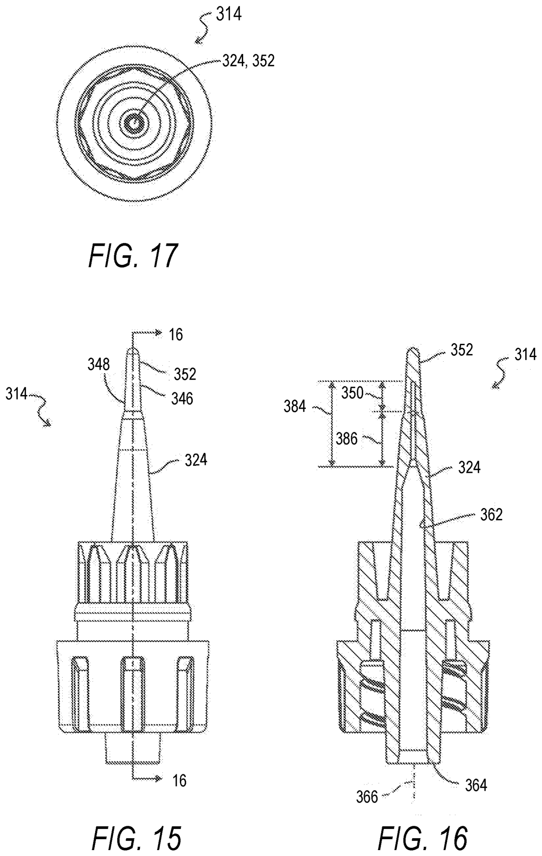

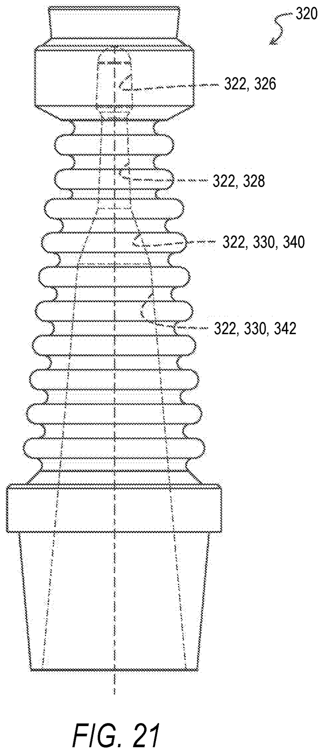

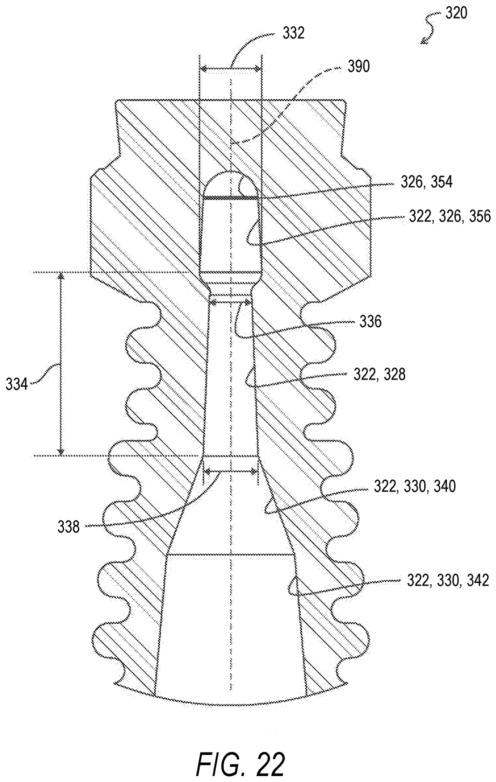

The details of construction of the first mating structure 314 are best shown in FIGS. 15-20. The details of construction of the second mating structure 316 are best shown in FIGS. 23 and 24. The details of construction of the resilient barrier 320 are best shown in FIGS. 21 and 22. FIG. 14 shows the injection port assembly 310 in an assembled cross section view with the first and second mating structures 314 and 316 coupled together and with the resilient barrier 320 received within the body 312 between the first and second mating structures 314 and 316.

As best seen in FIGS. 21 and 22, the resilient barrier 320 includes an internal cavity 322. It will be appreciated that the resilient barrier 320 is formed from an elastomeric material, and is shown in FIGS. 21 and 22 in its relaxed state in which the elastomeric material is relatively undeformed. It will also be appreciated that in FIG. 14 a hollow cannula 324 of the first mating structure 314 has been received in the internal cavity 322, thus deforming portions of the resilient barrier 320 radially outward so that the shape of the resilient barrier 320 as seen in FIG. 14, and particularly of its internal cavity 322, are different due to the resilient deformation thereof.

Referring now to FIGS. 21 and 22 which show the resilient barrier 320 and particularly its internal cavity 322 in their relaxed state, the internal cavity 322 in this relaxed state may be described as including a cavity nose portion 326, a cavity sealing portion 328, and a cavity guide portion 330.

As shown in FIG. 22, the cavity nose portion 326 has a cavity nose portion maximum inside diameter 332.

The cavity sealing portion 328 may be described as having a cavity sealing portion length 334. The cavity sealing portion 328 has a minimum cavity sealing portion inside diameter 336 at its upper end and is slightly tapered to a maximum cavity sealing portion inside diameter 338 at its lower end. It is noted that the cavity sealing portion 328 overall can be described as having a cavity sealing portion inside diameter smaller than the cavity nose portion maximum inside diameter 332 along at least a majority of the cavity sealing portion length 334. The cavity sealing portion inside diameter may be smaller than the cavity nose portion maximum inside diameter 332 along substantially the entire cavity sealing portion length 334.

The cavity sealing portion 328 of internal cavity 322 may be described as including a frusto-conical portion of increasing diameter in a proximal direction which increases from cavity sealing portion minimum inside diameter 336 to cavity sealing portion maximum inside diameter 338.

The cavity nose portion 326 may be described as being bulbous in shape as seen best in FIG. 22, and having a semi-spherical distal end 354. The cavity nose portion 326 may be further described as including a frusto-conical portion 356 of increasing diameter in a proximal direction from the semi-spherical distal end 354 to the cavity nose portion maximum inside diameter 332.

The cavity guide portion 330 is located on an opposite side of the cavity sealing portion 328 from the cavity nose portion 326. The cavity guide portion 330 tapers radially outward from the cavity sealing portion 328 and thus may be described as having a cavity guide portion inside diameter greater than the cavity sealing portion inside diameter 338. The cavity guide portion 330 may be further described as including a first frusto-conical portion 340 of increasing diameter in a proximal direction from the cavity sealing portion 38, and a second frusto-conical portion 42 adjacent the first frusto-conical portion 340, the second frusto-conical portion 342 having a smaller included angle than the first frusto-conical portion 340.

As previously noted, a hollow cannula 324 is coupled to the first mating structure 314, and in the example illustrated, the hollow cannula 324 is integrally formed with the first mating structure 314. The hollow cannula 324 is configured to be received within the resilient barrier 320 as shown for example in FIG. 14.

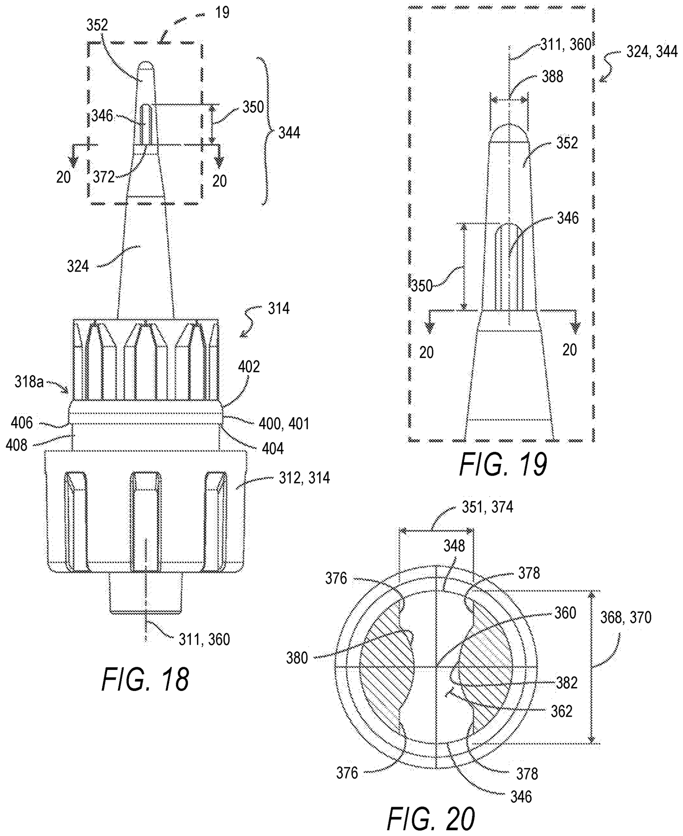

The hollow cannula 324 includes a distal end portion 344 shown in enlarged view in FIG. 19. The distal end portion 344 is configured to extend through the resilient barrier 320 when the resilient barrier 320 is in the more compressed second position. The cannula distal end portion 344 has at least one lateral outlet window 346 and in the example shown has a pair of lateral outlet windows 346 and 348.

As seen in FIGS. 18 and 19, each of the lateral outlet windows 346, 348 has a window length 350 which is less than the cavity sealing portion length 334. As best seen in FIG. 20, the windows 346 and 348 also have a width 351 perpendicular to the longitudinal central axis 311, 360 of the injection port assembly 310 and the cannula 324.

The cannula distal end portion 344 includes a cannula nose 352 located distally of the lateral outlet windows 346 and 348, and configured to be closely received in the cavity nose portion 326 of the resilient barrier 320 when the resilient barrier 320 is in the less compressed first position as shown in FIG. 14. The cannula nose may substantially fill the cavity nose portion when the resilient barrier is in the less compressed first position with the cannula nose closely received in the cavity nose portion. More particularly, the cannula nose may fill at least 90%, and more preferably at least 95%, of the cavity nose portion 396 by volume.

The cannula distal end portion 344 has a cannula distal end portion outside diameter both distally and proximally of the lateral outlet windows 346 and 348, which cannula distal end portion outside diameter is sufficiently greater than the respective inside diameters of the cavity sealing portion 328 of internal cavity 322 of resilient barrier 320 when the cannula nose 352 is received in the cannula nose portion 326 such that there is an interference fit between the cannula 324 and the resilient barrier 320. The interference fit extends along the lateral outlet windows 346 and 348 and both proximally and distally of the lateral outlet windows 346 and 348 so that the cavity sealing portion 328 of the resilient barrier 320 seals across the lateral outlet windows 346 and 348.

This is visualized in FIG. 14, wherein the relaxed position of the cavity sealing portion 328 of resilient barrier 320 is shown in dashed lines, and thus the extent of radially outward resilient deformation of the resilient barrier 320 by the cannula 324 received therein is readily apparent and it is apparent that this radially deformed portion of the cavity sealing portion 328 of resilient barrier 320 extends both distally and proximally from the lateral outlet windows 346 and 348.

The area between the dashed line relaxed state representation 328 and the solid line position of cavity sealing portion 328 as seen in FIG. 14 may be described as an interference fit 358 between the hollow cannula 324 and the resilient barrier 320. As is apparent in FIG. 14, this interference fit 358 between the cannula 324 and the resilient barrier 320 extends proximally into the first frusto-conical portion 340 of the cavity guide portion 330 of internal cavity 322 of resilient barrier 320. The interference fit 358 may also be described as a resilient interference zone spanning the length of the lateral windows 346 and 348.

At any one cross section along the axis 311 of injection port assembly 310, the interference fit 358 may be described as a radial interference which is mathematically determined by comparing the outside diameter of the cannula 324 to the inside diameter of the cavity sealing portion 328 in its relaxed state, and dividing that difference by two to provide the radial interference. Preferably the radial interference along the interference fit 358 is at least about 0.001 inch, optionally at least about 0.002 inch, optionally at least about 0.004 inch and optionally at least about 0.006 inch. Preferably the interference fit 358 between the cannula 324 and the resilient barrier 320 extends at least about 0.010 inch both proximally and distally from the lateral outlet windows 346 and 348.

### Non-Circular Cross Section Fluid Passageway

The hollow cannula 324 has a longitudinal central axis 360 which is coincident with the central axis 311 of the injection port assembly 310.

The cannula 324 has an internal fluid passageway 362 defined therein as best seen in FIG. 16. The internal fluid passageway 362 communicates the lateral outlet windows 346 and 348 with a proximal end of the first mating structure 314 which is configured to communicate with a fluid conduit 66 schematically illustrated in FIG. 16. The fluid conduit 366 may be representative of any structure to which the injection port assembly 310 is to be connected for fluid flow therewith.

FIG. 20 shows a downward facing cross section of the cannula 324 taken along line 20-20 of FIG. 18 and shows that the internal fluid passageway 362 has a non-circular cross section axially proximal from the windows 346 and 348. This non-circular cross section may be described as having a cross sectional area greater than a cross section area of a circle of diameter equal to the window width 351.

The two outlet windows 346 and 348 may be described as being diametrically opposed as is best seen in FIG. 20, and as being diametrically spaced apart by a window spacing 368. The non-circular cross section internal fluid passageway 362 as seen in FIG. 20 may be described as extending laterally to each of the two diametrically opposed outlet windows 346 and 348, and the non-circular cross section has a first lateral cross section dimension 370 at least equal to the window spacing 368 at an axial location immediately adjacent to a proximal end 372 of the windows 346 and 348.

The non-circular cross section of the internal fluid passageway 362 as seen in FIG. 20 immediately adjacent the proximal ends 372 of windows 346 and 348 may also be described as having a second lateral cross section dimension 374 which is at least equal to the window width 351. As is best visualized in FIG. 20, the non-circular cross section of the internal fluid passageway 362 may be described as being at least partially defined between first and second generally parallel opposed interior walls 376 and 378 of the hollow cannula 324.

The cannula 324 may further include first and second reinforcing ribs 380 and 382. The ribs 380 and 382 may be described as first and second diametrically opposed reinforcing ribs 380 and 382 extending radially inwardly from the first and second opposed interior walls 376 and 378, respectively.

This cross sectional shape of the internal fluid passageway 362 as visually depicted in FIG. 20 preferably extends along a non-circular cross section length 384 shown in FIG. 16. As is apparent in FIG. 16, the non-circular cross section length 384 extends along the window length 350 and continues proximally beyond the window length 350 into the internal passageway 362 of cannula 324 by a further distance 386 at least as long as the window length 350 and preferably longer than the window length 350.

Proximally of the non-circular cross sectional length 384, the internal passageway 362 may transition into a circular cross section extending to the proximal end 364 of the first mating structure 314.

The internal fluid passageway of non-circular cross section as depicted for example in FIG. 20 provides for increased fluid flow through the cannula 324 while maintaining the structural integrity of the cannula 324.

It will be appreciated by those skilled in the art that the typical dimensions of the cannula 324 are relatively small. For example, the cannula 324 may have an outside diameter 388 adjacent its distal end of approximately 0.04 inch, and the window width 351 may for example be approximately 0.026 inch. Thus if the internal fluid passageway 362 were of completely circular cross section as was typical in the prior art, a circular internal fluid passageway 362 leading to the lateral windows 346 and 348 would typically have a circular cross section with a diameter of about 0.026 inch. By constructing the cannula 324 with the non-circular cross sectional area depicted in FIG. 20 having a cross sectional area greater than a cross section of a circle of diameter equal to the window width 351, increased fluid flow through the cannula 324 for any given pressure of fluid supplied thereto is provided. Furthermore, due to the very small structures involved, the presence of the reinforcing ribs 380 and 382 aids in maintaining structural integrity of the tip portion of the cannula 324 around the windows 346 and 348, while still allowing this greater cross section internal passageway to be provided.

At least a portion of the hollow cannula 324, and preferably the entire hollow cannula 324 and the entire first mating structure 314, are constructed from an opaque material so that the internal fluid passageway 362 is not visible through the opaque cannula 324. For example, fluids such as blood contained in the internal fluid passageway 362, should not be visible through the opaque cannula 324. Furthermore, at least a portion of the resilient barrier 320 and at least a portion of the second mating structure 316 of body 312 are made from transparent materials so that the internal cavity 322 of the resilient barrier 320 and the exterior surface of cannula 324 are readily visible through the second mating structure 316 and the resilient barrier 320. Preferably the entire second mating structure 316 and the entire resilient barrier 320 are made from transparent material.

In a particular embodiment, at least a portion of the hollow cannula 324 is opaque, at least a portion of the second mating structure 316 is transparent, and at least a portion of the resilient barrier 320 is semi-transparent (e.g., translucent). Preferably, the entire hollow cannula 324 is a pastel opaque color, the entire second mating structure 316 is transparent, and the entire resilient barrier 320 is semi-transparent (e.g., translucent).

Thus any fluid leakage into the internal cavity 322 of resilient barrier 320 is readily visible through the resilient barrier 320 and the second mating structure 316 against the opaque background provided by the exterior surface of the opaque cannula 324.

Depending upon the fluids which are expected to be transmitted through the injection port assembly 310, the color of the opaque cannula 324 may be selected so as to provide the greatest contrast for ease of visualization of any fluids which might leak into the internal cavity 322 of resilient barrier 320. For example, in certain embodiments, the pastel color is selected from the group consisting of light blue, blue, green, and turquoise. In one particular embodiment, the cannula 324 may be colored a light pastel blue, which provides a distinct contrast with any blood leakage into the internal cavity 322.

### Improved Snap Lock Feature

The snap lock feature 318 is improved over prior designs so as to provide a substantial increase in the tension force required to pull the first and second mating structures 314 and 316 apart after assembly.

Referring to FIG. 18 the first mating structure 314 includes a first locking portion 318a defined by a snap lock ring 400 having an outermost surface 401 defined between a tapered upper guiding surface 402 and a locking shoulder 404. The locking shoulder 404 is at substantially 90 degrees to the outer surface 401 thus defining a relatively sharp locking edge 406. Located below the snap lock ring 400 is a stabilizing ring shelf 408.

The second mating structure 316 includes a second locking portion 318b best seen in FIG. 24. The second locking portion 318b includes a snap lock ring channel 410 in which the snap lock ring 400 is to be received. Located below the snap lock ring channel 410 is a plurality of stabilizing ring securement segments 412 separated by gaps 414. It can be seen that the snap lock ring channel 410 is curved in cross-section and forms a curved tapered upper locking surface 416 on each of the stabilizing ring securement segments 412.

When the first and second mating structures 314 and 316 are snapped together as seen in FIG. 14. The sharp locking edge 406 of the snap lock ring 400 bites into the curved tapered upper locking surfaces 416 of the stabilizing ring securement segments 412 to securely prevent the first and second mating structures 314 and 316 from being pulled back apart.

When the current design is compared to a snap lock feature like that shown in U.S. Patent Application Publication No. 2016/0129235 wherein the engaging surfaces of the snap lock ring and of the stabilizing ring securement segments are both tapered at complementary angles, a substantial increase in the force required to pull apart the first and second mating structures is provided. The required pull apart force was increased from about 14 pounds with the design of U.S. Patent Application Publication No. 2016/0129235 to about 54 pounds with the present design. The snap lock feature 318 can be described as having the first and second locking portions 318a and 318b configured such that a force of at least 30 pounds, and more preferably at least 40 pounds, is required to pull apart the first and second mating structures 314 and 316.

### Improved Performance

The provision of the interference fit 358 between the cannula 324 and the resilient barrier 320 of FIGS. 14-24, and of the cannula 124 and resilient barrier 120 of the embodiment of FIGS. 6-11, has provided substantially increased resistance to leaking due to back pressure within the injection port assemblies 10 and 110 as compared to a similar prior design of the assignee of the present invention as depicted in U.S. Patent Application Publication No. 2016/0129235.

For example, using the embodiment of FIGS. 6-11, tests were run on back pressure resistance, flow rate and fluid displacement.

Average back pressure resistance has improved from 47 psi with the previous design to over 68 psi with the design depicted herein having the interference fit. Testing was done using standardized procedures wherein each sample was submerged in water and subjected to increased pressure until air bubbles were observed leaking from the submerged sample.

Average fluid flow rates at gravity increased from 48 mL/min for a similar design having a circular cross section internal fluid passageway, up to an average of approximately 139 mL/min for the cross sectional area generally like that shown in FIG. 6A-6C. In these tests for a lot of 59 samples, flow rates ranged from a minimum of 127 mL/min to a max of 155 mL/min for sterilized samples, and from a minimum of 127 mL/min to a max of 163 mL/min for non-sterilized samples

Additionally, fluid reflux was measured at 0.00 mL with the embodiment of FIGS. 6-11.

### Methods of Use

As depicted in FIGS. 14 and 10 for the respective embodiments, the upper and lower parts 316, 116 and 314, 114 of the bodies 312, 112 are assembled with the resilient barriers 320, 120 contained therein and with the cannula 324, 124 received with the internal cavity 322, 122 of respective resilient barrier 320, 120. It will be appreciated that in the assembled arrangement as seen in FIGS. 14 and 10, there may be a slight axial compression of the resilient barrier 320, 120 from its completely relaxed state. The position of the resilient barrier 320, 120 as depicted in FIGS. 14 and 10 may be described as a less axially compressed first position in which fluid flow through the injection port assembly 310, 110 is blocked. It will be appreciated that the distal end of the resilient barrier 320, 120 has a precut slit 390, 190 formed therein through which the distal end portion 344, 144 of cannula 324, 124 will protrude when the resilient barrier 320, 120 is moved to its more axially compressed second position like that shown in FIG. 11.

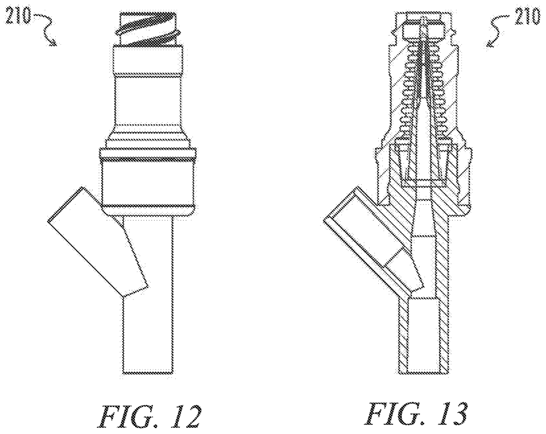

The injection port assembly 310, 110 may be connected to various conduits and medical devices so as to provide for intravenous injection into the patient's body and for collection of blood samples from the patient. The injection port assembly 310 may be incorporated into an IV pump set or IV administration set in a Y-site injection port configuration. FIGS. 12 and 13 for example, show a Y-site injection port arrangement 210 utilizing the three window embodiment of FIGS. 6-11.

As depicted in FIGS. 10 and 11, the resilient barrier 120 may be moved from its closed first position to its open second position by engagement of the injection port assembly 110 by a male-luer slip syringe 92. Beginning in the closed position of FIG. 10, as is indicated by the arrow 94 the syringe 92 is pushed downward engaging the distal end of the resilient barrier 120 and forcing it downward relative to the cannula 124 so as to expose the distal end portion 144 of cannula 124 thus allowing the lateral windows such as 146, 147 and 148 to communicate with the interior 96 of syringe 92. This allows fluids to be injected into or withdrawn from the patient's blood stream.

The resilient barrier 320, 120 may for example be formed of a silicone rubber material having a diameter in the range of from about 50 to about 70, and preferably having a diameter of about 60. The silicone rubber material may have a small amount of phenyl oil included therein to provide an internal lubricant when the resilient barrier 320, 120 slides along the outer surface of the cannula 324, 124. The exterior surface of cannula 324, 124 may be treated to form a slightly roughened surface with irregularities on the order of 0.001 inch and may be lubricated with silicone oil to further aid in the movement of the resilient barrier 320 between its closed and open positions of FIGS. 10 and 11. These features aid in allowing the resilient barrier 320, 120 to substantially instantaneously snap back from its open position of FIG. 11 to its closed position of FIG. 10 upon removal of the syringe 92.

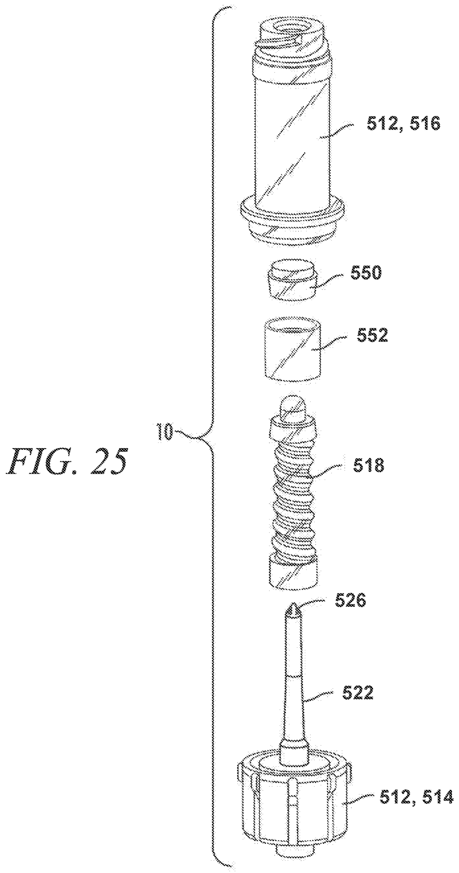

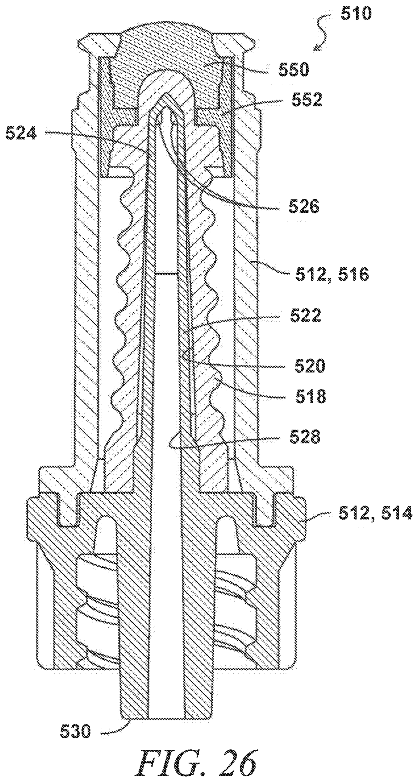

### The Embodiment Of Figures 25 and 26:

Figures 25 and 26 show an alternative embodiment of an injection port assembly incorporating the present invention, which is generally designated by the number 510. The general construction of the components of the injection port assembly 510 and the operation thereof, but not the transparency or opaqueness of the materials from which it is constructed, are generally in accordance with the teachings of U.S. Patent No. 8,096,525 to Ryan, the details of which are incorporated herein by reference.

The injection port assembly 510 includes a body 512 having a first mating structure 514 and a second mating structure 516 configured to be coupled to the first mating structure 514.

A resilient barrier 518 is configured to be received within the body 512 and is compressible from a less compressed first position in which fluid flow through the injection port assembly 510 is blocked, to a more compressed second position in which fluid flow through the injection port assembly 510 is permitted. The resilient barrier 518 has an internal cavity 520.

A hollow cannula 522 is coupled to the first mating structure 514 and configured to be received within the resilient barrier 518. The hollow cannula 522 has a cannula distal end portion 524 configured to extend through the resilient barrier 518 when the resilient barrier 518 is in the more compressed second position. The cannula distal end portion 524 has at least one outlet window 526 defined therein.

The hollow cannula 522 is preferably integrally formed with the first mating structure 514. Hollow cannula 522 has an internal fluid passageway 528 defined therein, communicating a proximal end 530 of the first mating structure 514 with the outlet windows 526.

At least a portion of the hollow cannula 522, and preferably the entire hollow cannula 522 and the entire first mating structure 514, are constructed from an opaque material so that the internal fluid passageway 528 is not visible through the opaque cannula 522. Furthermore, at least a portion of the resilient barrier 518 and at least a portion of the second mating structure 516 of body 512 are made from transparent materials so that the internal cavity 520 of the resilient barrier 518 and the exterior surface of cannula 522 are readily visible through the second mating structure 516 and the resilient barrier 518. Preferably the entire second mating structure 516 and the entire resilient barrier 518 are made from transparent material.

The injection port assembly 510 also includes a separate septum 550 and an H-guide 552 which are assembled with the upper end of the resilient barrier 518 as generally seen in Fig. 26. The H-guide has an "H" shape cross-section. The H-guide 552 is connected to the separate septum 550 and the resilient barrier 518 to guide the end of the resilient barrier 518 and the separate septum 550 in sliding motion within the second mating structure 516. Although it is preferable that the separate septum 550 and the H-guide 552 are also constructed of a clear material, it is not required. It will be appreciated that even if the separate septum 550 and/or the H-guide 552 are constructed of opaque material, at least a portion of the internal cavity 520 of the resilient barrier 518 will be visible through the resilient barrier 518 and the second mating structure 516.

Thus it is seen that the apparatus of the present invention readily achieves the ends and advantages mentioned as well as those inherent therein. While certain preferred embodiments of the invention have been illustrated and described for purposes of the present disclosure, numerous changes in the arrangement and construction of parts and steps may be made by those skilled in the art, which changes are encompassed within the scope and spirit of the present invention as defined by the appended claims.

### Further Embodiments:

In a first aspect, this disclosure provides an injection port assembly comprising: a body having a first mating structure and a second mating structure configured to be coupled to the first mating structure; a resilient barrier configured to be received within the body and compressible from a less compressed first position in which fluid flow through the injection port assembly is blocked to a more compressed second position in which fluid flow through the injection port assembly is permitted, the resilient barrier including an internal cavity; a hollow cannula coupled to the first mating structure and configured to be received within the resilient barrier, the hollow cannula having a cannula end portion configured to extend through the resilient barrier when the resilient barrier is in the more compressed second position, the cannula end portion having at least one outlet window; wherein at least a portion of the hollow cannula is opaque and at least a portion of both the second mating structure and the resilient barrier are transparent.

In certain embodiments of the first aspect, fluid leakage into the internal cavity of the resilient barrier is visible though the resilient barrier and the second mating structure against an opaque background provided by the opaque cannula.

In certain embodiments of the first aspect, the opaque portion of the cannula has a color lighter than blood.

In certain embodiments of the first aspect, the opaque portion of the cannula has a color other than red.

In certain embodiments of the first aspect, the opaque portion of the cannula has a pastel color such as to contrast to blood in the internal cavity of the resilient barrier.

In certain embodiments of the first aspect, the opaque portion of the cannula is colored a light pastel blue.

In certain embodiments of the first aspect, the opaque portion of the cannula is integrally formed with the first mating structure and the entire first mating structure is opaque.

In certain embodiments of the first aspect, the hollow cannula has an internal fluid passageway, and blood inside the internal fluid passageway is not visible through the opaque portion of the cannula.

In certain embodiments of the first aspect, the hollow cannula has an internal fluid passageway and the internal fluid passageway is not visible through the opaque portion of the cannula.

In certain embodiments of the first aspect, at least a portion of the internal cavity of the resilient barrier is visible through the resilient barrier and the second mating structure.

In certain embodiments of the first aspect, the entire cannula is opaque and the entire second mating structure and the entire resilient barrier are transparent.

In certain embodiments of the first aspect, the injection port assembly further comprises: a separate septum engaging an end of the resilient barrier; a guide connected to the separate septum and the resilient barrier to guide the end of the resilient barrier and the separate septum in sliding motion within the second mating structure; wherein the separate septum and the guide are both transparent.

In a second aspect, this disclosure provides an injection port assembly, comprising: a body having a first mating structure and a second mating structure configured to be coupled to the first mating structure; a resilient barrier configured to be received within the body and compressible from a less compressed first position in which fluid flow through the injection port assembly is blocked to a more compressed second position in which fluid flow through the injection port assembly is permitted, the resilient barrier including an internal cavity; a hollow cannula coupled to the first mating structure and configured to be received within the internal cavity of the resilient barrier, the hollow cannula having a cannula end portion configured to extend through the resilient barrier when the resilient barrier is in the more compressed second position, the cannula having an internal fluid passageway; wherein at least a portion of the hollow cannula is opaque so that the internal fluid passageway of the cannula is not visible through the opaque portion of the cannula; and wherein at least a portion of both the second mating structure and the resilient barrier are transparent so that at least a portion of the internal cavity of the resilient barrier is visible though the resilient barrier and the second mating structure.

In certain embodiments of the second aspect, the opaque portion of the cannula has a color lighter than blood.

In certain embodiments of the second aspect, the opaque portion of the cannula has a color other than red.

In certain embodiments of the second aspect, the opaque portion of the cannula has a pastel color such as to contrast to any blood which leaks into the internal cavity of the resilient barrier.

In certain embodiments of the second aspect, the opaque portion of the cannula is colored a light pastel blue.

In certain embodiments of the second aspect, the opaque portion of the cannula is integrally formed with the first mating structure and the entire first mating structure is opaque.

In certain embodiments of the second aspect, any blood inside the internal fluid passageway of the hollow cannula is not visible through the opaque portion of the cannula.

In certain embodiments of the second aspect, the entire cannula is opaque and the entire second mating structure and the entire resilient barrier are transparent.

In certain embodiments of the second aspect, the injection port assembly further comprises: a separate septum engaging an end of the resilient barrier; a guide connected to the separate septum and the resilient barrier to guide the end of the resilient barrier and the separate septum in sliding motion within the second mating structure; wherein the separate septum and the guide are both transparent.

In a third aspect, this disclosure provides an injection port assembly, comprising: a body having a first mating structure and a second mating structure configured to be coupled to the first mating structure; a resilient barrier configured to be received within the body and compressible from a less compressed first position in which fluid flow through the injection port assembly is blocked to a more compressed second position in which fluid flow through the injection port assembly is permitted, the resilient barrier including an internal cavity, the internal cavity when the resilient barrier is in a relaxed state including: a cavity nose portion having a cavity nose portion maximum inside diameter; a cavity sealing portion having a cavity sealing portion length, the cavity sealing portion having a cavity sealing portion inside diameter smaller than the cavity nose portion maximum inside diameter along at least a majority of the cavity sealing portion length; and a cavity guide portion located on an opposite side of the cavity sealing portion from the cavity nose portion, the cavity guide portion having a cavity guide portion inside diameter greater than the cavity sealing portion inside diameter; and a hollow cannula coupled to the first mating structure and configured to be received within the resilient barrier, the hollow cannula having a cannula distal end portion configured to extend through the resilient barrier when the resilient barrier is in the more compressed second position, the cannula distal end portion having at least one lateral outlet window having a window length less than the cavity sealing portion length, the cannula distal end portion including a cannula nose located distally of the lateral outlet window and configured to be closely received in the cavity nose portion of the resilient barrier when the resilient barrier is in the less compressed first position, the cannula distal end portion both distally and proximally of the lateral outlet window having a cannula distal end portion outside diameter sufficiently greater than the cavity sealing portion inside diameter such that when the cannula is received in the resilient barrier with the cannula nose received in the cavity nose portion there is an interference fit between the cannula and the resilient barrier extending along the lateral outlet window and both proximally and distally of the lateral outlet window so that the cavity sealing portion of the resilient barrier seals across the lateral outlet window.

In certain embodiments of the third aspect, the cavity nose portion is bulbous in shape and has a semi-spherical distal end, and the cavity nose portion includes a frusto-conical portion of increasing diameter in a proximal direction from the semi-spherical distal end to the cavity nose portion maximum inside diameter.

In certain embodiments of the third aspect, the cavity sealing portion includes a frusto-conical portion of increasing diameter in a proximal direction from a cavity sealing portion minimum inside diameter to a cavity sealing portion maximum inside diameter.

In certain embodiments of the third aspect, the cavity guide portion includes a frusto-conical portion of increasing diameter in a proximal direction from the cavity sealing portion.

In certain embodiments of the third aspect, the interference fit between the cannula and the resilient barrier extends into the frusto-conical portion of the cavity guide portion.

In certain embodiments of the third aspect, the cavity guide portion includes a first frusto-conical portion of increasing diameter in a proximal direction adjacent the cavity sealing portion and a second frusto-conical portion adjacent the first frusto-conical portion, the second frusto-conical portion having a smaller included angle than the first frusto-conical portion.

In certain embodiments of the third aspect, the interference fit between the cannula and the resilient barrier extends at least about 0.010 inch proximally and distally of the outlet window.

In certain embodiments of the third aspect, the cannula and the resilient barrier are configured such that interference fit provides at least about 0.001 inch radial interference between the cannula and the resilient barrier.

In certain embodiments of the third aspect, the cannula and the resilient barrier are configured such that interference fit provides at least about 0.002 inch radial interference between the cannula and the resilient barrier.

In certain embodiments of the third aspect, the cannula and the resilient barrier are configured such that interference fit provides at least about 0.004 inch radial interference between the cannula and the resilient barrier.

In certain embodiments of the third aspect, the cannula nose substantially fills the cavity nose portion when the resilient barrier is in the less compressed first position with the cannula nose closely received in the cavity nose portion.

In certain embodiments of the third aspect, when the resilient barrier is in the relaxed state the cavity sealing portion inside diameter is smaller than the cavity nose portion maximum inside diameter along the entire cavity sealing portion length.

In certain embodiments of the third aspect, the at least one lateral outlet window comprises two diametrically opposed outlet windows.

In certain embodiments of the third aspect, the at least one lateral outlet window comprises three circumferentially equally spaced outlet windows
In a fourth aspect, this disclosure provides an injection port assembly, comprising: a body having a first mating structure and a second mating structure configured to be coupled to the first mating structure; a resilient barrier configured to be received within the body and compressible from a less compressed first position in which fluid flow through the injection port assembly is blocked to a more compressed second position in which fluid flow through the injection port assembly is permitted, the resilient barrier including an internal cavity; and a hollow cannula coupled to the first mating structure and configured to be received within internal cavity of the resilient barrier, the hollow cannula having a longitudinal central axis, the hollow cannula including: a cannula distal end portion configured to extend through the resilient barrier when the resilient barrier is in the more compressed second position; at least one lateral outlet window formed in the cannula distal end portion, the at least one lateral outlet window having a window width perpendicular to the longitudinal central axis; and an internal fluid passageway defined in the hollow cannula and configured to communicate the at least one lateral outlet window with a fluid conduit connected to the first mating structure, the internal fluid passageway having a non-circular cross section axially proximal from the window, the non-circular cross section having a cross section area greater than a cross section area of a circle of diameter equal to the window width.

In certain embodiments of the fourth aspect, the at least one lateral outlet window comprises two diametrically opposed outlet windows. In some such embodiments, the two diametrically opposed outlet windows are diametrically spaced apart by a window spacing.

In certain embodiments of the fourth aspect, the internal fluid passageway extends laterally to each of the two diametrically opposed outlet windows and the non-circular cross section has a first lateral cross section dimension at least equal to the window spacing immediately adjacent a proximal end of the windows. In some such embodiments, the non-circular cross section immediately adjacent the proximal ends of the windows has a second lateral cross section dimension perpendicular to the first lateral cross section dimension, which second lateral cross section dimension is at least equal to the window width.

In certain embodiments of the fourth aspect, the non-circular cross section of the internal fluid passageway is at least partially defined between first and second generally parallel opposed interior walls of the hollow cannula.

In certain embodiments of the fourth aspect, the at least one lateral outlet window comprises three circumferentially equally spaced lateral outlet windows.

In some such embodiments, the non-circular cross section of the internal fluid passageway is a three lobed cross section.

In some such embodiments, the three windows each have a window length; and the three lobed cross section tapers radially outward and extends proximally from the windows for a distance at least as long as the window length.

In certain embodiments of the fourth aspect, the at least one window has a window length; and the non-circular cross section of the internal fluid passageway extends proximally beyond the length of the window further into the internal fluid passageway by a further distance at least as long as the window length.

In a fifth aspect, this disclosure provides an injection port assembly, comprising: a body having a first mating structure and a second mating structure configured to be coupled to the first mating structure; a resilient barrier configured to be received within the body and compressible from a less compressed first position in which fluid flow through the injection port assembly is blocked to a more compressed second position in which fluid flow through the injection port assembly is permitted, the resilient barrier including an internal cavity; a hollow cannula coupled to the first mating structure and configured to be received within internal cavity of the resilient barrier; wherein the first mating structure includes a first locking portion and the second mating structure includes a second locking portion, and the first and second locking portions are configured to lock together as the second mating structure is coupled to the first mating structure; wherein one of the first and second locking portions includes a locking edge and the other of the first and second locking portions includes a tapered locking surface, the locking edge being configured to engage the tapered locking surface to resist disengagement of the first mating structure from the second mating structure.

In certain embodiments of the fifth aspect, the locking edge is defined by a substantially 90 degree corner.

In certain embodiments of the fifth aspect, the tapered locking surface is a curved tapered locking surface.

In certain embodiments of the fifth aspect, the tapered locking surface is defined on the second locking portion of the second mating structure, and the tapered locking surface is a segmented surface defined on a plurality of stabilizing ring securement segments of the second mating structure.

In certain embodiments of the fifth aspect, the first and second locking portions are configured such that a force of at least 30 pounds is required to pull apart the first and second mating structures.

In certain embodiments of the fifth aspect, the first and second locking portions are configured such that a force of at least 40 pounds is required to pull apart the first and second mating structures.

In a sixth aspect, this disclosure provides an injection port assembly, comprising a body having a first mating structure and a second mating structure configured to be coupled to the first mating structure; a resilient barrier configured to be received within the body and compressible from a less compressed first position in which fluid flow through the injection port assembly is blocked to a more compressed second position in which fluid flow through the injection port assembly is permitted, the resilient barrier including an internal cavity, the internal cavity when the resilient barrier is in a relaxed state including: a bulbous cavity nose portion having a cavity nose portion maximum inside diameter and a semi-spherical distal end, the bulbous cavity nose portion including a frusto-conical portion of increasing diameter in a proximal direction from the semi-spherical distal end to the cavity nose portion maximum inside diameter; and a hollow cannula coupled to the first mating structure and configured to be received within the resilient barrier, the hollow cannula having a cannula distal end portion configured to extend through the resilient barrier when the resilient barrier is in the more compressed second position, the cannula distal end portion having at least one lateral outlet window; wherein at least a portion of the hollow cannula is opaque and at least a portion of both the second mating structure and the resilient barrier are transparent.

In certain embodiments of the sixth aspect, the opaque portion of the cannula has a pastel color such as to contrast to any blood which leaks into the internal cavity of the resilient barrier. In some such embodiments, the pastel color is selected from the group consisting of light blue, blue, green, and turquoise.

In certain embodiments of the sixth aspect, the opaque portion of the cannula is integrally formed with the first mating structure and the entire first mating structure is opaque.

In certain embodiments of the sixth aspect, fluid leakage into the internal cavity of the resilient barrier is visible through the resilient barrier and the second mating structure against an opaque background provided by the opaque cannula.

In certain embodiments of the sixth aspect, the hollow cannula has an internal fluid passageway, and the internal fluid passageway and/or any blood inside the internal fluid passageway of the hollow cannula is not visible through the opaque portion of the cannula.

In certain embodiments of the sixth aspect, the internal cavity, when the resilient barrier is in a relaxed state, further comprises a cavity sealing portion having a cavity sealing portion length, the cavity sealing portion having a cavity sealing portion inside diameter smaller than the cavity nose portion maximum inside diameter along at least a majority of the cavity sealing portion length. In some such embodiments, the at least one lateral outlet window has a window length less than the cavity sealing portion length. In some such embodiments, the internal cavity, when the resilient barrier is in a relaxed state, further comprises a cavity guide portion located on an opposite side of the cavity sealing portion from the cavity nose portion, the cavity guide portion having a cavity guide portion inside diameter greater than the cavity sealing portion inside diameter. In some such embodiments, the cannula distal end portion includes a cannula nose located distally of the lateral outlet window and configured to be closely received in the cavity nose portion of the resilient barrier when the resilient barrier is in the less compressed first position, the cannula distal end portion both distally and proximally of the lateral outlet window having a cannula distal end portion outside diameter sufficiently greater than the cavity sealing portion inside diameter such that when the cannula is received in the resilient barrier with the cannula nose received in the cavity nose portion there is an interference fit between the cannula and the resilient barrier extending along the lateral outlet window and both proximally and distally of the lateral outlet window so that the cavity sealing portion of the resilient barrier seals across the lateral outlet window.

In a seventh aspect, this disclosure provides an injection port assembly, comprising a body having a first mating structure and a second mating structure configured to be coupled to the first mating structure; a resilient barrier configured to be received within the body and compressible from a less compressed first position in which fluid flow through the injection port assembly is blocked to a more compressed second position in which fluid flow through the injection port assembly is permitted, the resilient barrier including an internal cavity, the internal cavity when the resilient barrier is in a relaxed state including: a cavity nose portion having a cavity nose portion maximum inside diameter; a cavity sealing portion having a cavity sealing portion length, the cavity sealing portion having a cavity sealing portion inside diameter smaller than the cavity nose portion maximum inside diameter along at least a majority of the cavity sealing portion length; and a cavity guide portion located on an opposite side of the cavity sealing portion from the cavity nose portion, the cavity guide portion having a cavity guide portion inside diameter greater than the cavity sealing portion inside diameter; and a hollow cannula coupled to the first mating structure and configured to be received within the resilient barrier, the hollow cannula having a cannula distal end portion configured to extend through the resilient barrier when the resilient barrier is in the more compressed second position, the cannula distal end portion having at least one lateral outlet window having a window length less than the cavity sealing portion length, the cannula distal end portion including a cannula nose located distally of the lateral outlet window and configured to be closely received in the cavity nose portion of the resilient barrier when the resilient barrier is in the less compressed first position, the cannula distal end portion both distally and proximally of the lateral outlet window having a cannula distal end portion outside diameter sufficiently greater than the cavity sealing portion inside diameter such that when the cannula is received in the resilient barrier with the cannula nose received in the cavity nose portion there is an interference fit between the cannula and the resilient barrier extending along the lateral outlet window and both proximally and distally of the lateral outlet window so that the cavity sealing portion of the resilient barrier seals across the lateral outlet window; wherein the cannula nose substantially fills the cavity nose portion when the resilient barrier is in the less compressed first position with the cannula nose closely received in the cavity nose portion; wherein at least a portion of the hollow cannula is opaque and at least a portion of both the second mating structure and the resilient barrier are transparent.

In certain embodiments of the seventh aspect, the opaque portion of the cannula has a pastel color such as to contrast to any blood which leaks into the internal cavity of the resilient barrier. In some such embodiments, the pastel color is selected from the group consisting of light blue, blue, green, and turquoise.

In certain embodiments of the seventh aspect, the opaque portion of the cannula is integrally formed with the first mating structure and the entire first mating structure is opaque.

In certain embodiments of the seventh aspect, fluid leakage into the internal cavity of the resilient barrier is visible through the resilient barrier and the second mating structure against an opaque background provided by the opaque cannula.

In certain embodiments of the seventh aspect, the hollow cannula has an internal fluid passageway, and the internal fluid passageway and/or any blood inside the internal fluid passageway of the hollow cannula is not visible through the opaque portion of the cannula.

In certain embodiments of the seventh aspect, the interference fit between the cannula and the resilient barrier extends at least about 0.010 inch proximally and distally of the outlet window.

In certain embodiments of the seventh aspect, the cannula and the resilient barrier are configured such that interference fit provides at least about 0.001 inch radial interference between the cannula and the resilient barrier.

In certain embodiments of the seventh aspect, the cannula and the resilient barrier are configured such that interference fit provides at least about 0.002 inch radial interference between the cannula and the resilient barrier.

In certain embodiments of the seventh aspect, the cannula and the resilient barrier are configured such that interference fit provides at least about 0.004 inch radial interference between the cannula and the resilient barrier.

## Claims

1. An injection port assembly, comprising:
a body having a first mating structure and a second mating structure configured to be coupled to the first mating structure;
a resilient barrier configured to be received within the body and compressible from a less compressed first position in which fluid flow through the injection port assembly is blocked to a more compressed second position in which fluid flow through the injection port assembly is permitted, the resilient barrier including an internal cavity;
a hollow cannula coupled to the first mating structure and configured to be received within internal cavity of the resilient barrier;
wherein the first mating structure includes a first locking portion and the second mating structure includes a second locking portion, and the first and second locking portions are configured to lock together as the second mating structure is coupled to the first mating structure;
wherein one of the first and second locking portions includes a locking edge and the other of the first and second locking portions includes a tapered locking surface, the locking edge being configured to engage the tapered locking surface to resist disengagement of the first mating structure from the second mating structure.

2. The injection port assembly of claim 1, wherein:
the locking edge is defined by a substantially 90 degree corner.

3. The injection port assembly of claim 1 or 2, wherein:
the tapered locking surface is a curved tapered locking surface.

4. The injection port assembly of claim 3, wherein:
the tapered locking surface is defined on the second locking portion of the second mating structure, and the tapered locking surface is a segmented surface defined on a plurality of stabilizing ring securement segments of the second mating structure.

5. The injection port assembly of any of the preceding claims, wherein:
the first and second locking portions are configured such that a force of at least 30 pounds is required to pull apart the first and second mating structures.

6. The injection port assembly of any of the preceding claims, wherein:
the first and second locking portions are configured such that a force of at least 40 pounds is required to pull apart the first and second mating structures.
